# EUROPEAN PATENT APPLICATION

(11) **EP 4 245 742 A1**
(43) Date of publication of application: **20.09.2023**
(21) Application number: 22162109.7
(22) Date of filing: 15.03.2022
(51) Int. Cl.: C07B 59/00, C07F 9/54, C07C 17/02, C07C 17/16, C07C 19/01, C07F 9/00, C07C 1/34

(54) **13C-LABELLED CHLORINATED PARAFFINS AND THEIR PREPARATION**

(71) Applicant: Chiron AS, 7041 Trondheim (NO)
(72) Inventor: GOROVOY, Alexey, 7020 Trondheim (NO); LIU Huiling, 7049 Trondheim (NO); TUMA, Jiri, 27201 Kladno (CZ); VALDERHAUG, Solveig, 7023 Trondheim (NO); NYGREN, Jonatan, 7067 Trondheim (NO); JOHANSEN, Jon Eigill, 7092 Tiller (NO)
(74) Representative: Rupprecht, Kay

(57) **Abstract**

The present invention is directed to ¹³C-labelled chlorinated paraffins, preferably having neither geminal nor terminal chlorine atoms in their structure, processes for their preparation as well as their uses.

## Description

The present invention is directed to ¹³C-labelled chlorinated paraffins and processes for their preparation as well as their uses.

Any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as set forth herein supersedes any conflicting material incorporated herein by reference. Any material, or portion thereof, that is said to be incorporated by reference herein, but which conflicts with existing definitions, statements, or other disclosure material set forth herein is only incorporated to the extent that no conflict arises between that incorporated material and the existing disclosure material.

### Prior art:

Chlorinated paraffins (CP or CPs) are a class of industrial chemicals used as high-temperature lubricants in metal-working machinery and as flame-retardant plasticizers in vinyl plastics. Less common applications include the use as flame retardants in rubber, paints, adhesives and as sealants. Technical CPs are complex mixtures consisting of polychlorinated alkanes (PCAs) with a chlorination degree between 30% and 70% and a linear alkane chain with length of C8-C9 (very short-chain CPs, vSCCPs), C₁₀-C₁₃ (short-chain CPs, SCCPs), C₁₄-C₁₇ (medium-chain CPs, MCCPs), and C₁₈-C₃₀ (long-chain CPs, LCCPs). A general molecule formula of CPs is: CₙH_{2n+2-y}Cl_{y}, n=10-30

The industrial chemicals of CPs are produced by chlorination of C₁₀-C₃₀ n-alkanes using molecular chlorine, either of the liquid paraffin or in a solvent. Depending on the n-alkane feedstock, the reaction takes place at temperatures between 50 and 150°C, at elevated pressures and/or in the presence of UV light. The total global production remains largely unknown but is believed to exceed at least two million metric tonnes per year.

CPs show resistance to degradation, and some show bioaccumulation and toxic potential, and are suspected to be carcinogenic to humans. SCCPs have been prohibited by the POP Regulation in the EU since 2017 and placed on several monitoring lists such as the EU Water Framework Directive.

CPs need to be continuously monitored in environment and food and feed matrices. CPs are analysed by GC coupled to either a high-resolution or low-resolution MS (HRMS or LRMS). HRMS, two dimensional GC (GCxGC), or LC-HRMS (APCI-QTOF-HRMS) is the state-of-art to distinguish SCCPs, MCCPs and LCCPSs.

One of the main challenges in CPs analysis or determination is the lack of suitable standards and matrix reference materials. So far there are no suitable and generally accepted reference standards commercially available yet.

### Objects of the invention:

It was an object of the present invention to provide novel ¹³C-labelled chlorinated paraffins. These should preferably be useable in the analysis or determination,
particularly as internal standards, of CP.

It was a further object of the invention to provide novel methods/processes for the preparation of such ¹³C-labelled chlorinated paraffins.

Additionally, it was an object of the present invention to provide for uses of the novel ¹³C-labelled chlorinated paraffins and those prepared with the methods/processes of the present invention.

Further objects will become apparent to those skilled in the art upon regarding the following disclosure.

### Solution of the Invention:

These and other objects are solved by the matter outlined in the appended independent claims.

Preferred embodiments are outlined in the dependent claims as well as the following description.

### Detailed description:

It should be understood that this invention is not limited to the embodiments disclosed in this summary, or the description that follows, but is intended to cover modifications that are within the spirit and scope of the invention, as de-fined by the claims.

In the present application, including the claims, other than in the operating examples or where otherwise indicated, all numbers expressing quantities or characteristics are to be understood as being modified in all instances by the term "about". Accordingly, unless indicated to the contrary, any numerical parameters set forth in the following description may vary depending on the desired properties one seeks to obtain in the compositions and methods according to the present disclosure. At the very least, and not as an attempt to limit the application of the doctrine of equivalents to the scope of the claims, each numerical parameter described in the present description should at least be construed in light of the number of reported significant digits and by applying ordinary rounding techniques.

Also, it should be understood that any numerical range recited herein is in-tended to include all sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10. The terms "one," "a," or "an" as used herein are intended to include "at least one" or "one or more," unless otherwise indicated.

In the present invention, unless otherwise specified, the reactions are conducted at room temperature, which is understood as being 20°C.

In the present invention, unless otherwise specified, the reactions are conducted at ambient pressure, which is understood as being 101,325 kPa.

In the present invention the term "fatty acid" is used as in the IUPAC Gold Book as: "Aliphatic monocarboxylic acids derived from or contained in esterified form in an animal or vegetable fat, oil or wax. Natural fatty acids commonly have a chain of 4 to 28 carbons (usually unbranched and even-numbered), which may be saturated or unsaturated.".

The present invention in a first embodiment is directed to a process for preparing ¹³C-labelled chlorinated paraffins.

While with this process in principle any ¹³C-labelled chlorinated paraffins can be prepared, in the present invention it is preferred for preparing ¹³C-labelled chlorinated paraffins having neither geminal nor terminal chlorine atoms in their structure. This process of the present invention comprises or consists of the steps of:
V) providing an alkene
   - having one or more alpha,beta-ethylenically unsaturated double bonds in its main chain,
   - one or more carbon atoms in its main chain being replaced by ¹³C-atom(s),
   - and optionally one chlorine atom or an odd number of chlorine atoms in its main chain, with the proviso that neither geminal nor terminal chlorines are present and none are present on an atom that is part of an alpha,beta-ethylenically unsaturated double bond,
      or a mixture of such alkenes,
VI) reacting the alkene or mixture of alkenes with a trichloride, a tribromide or triiodide, preferably a trichloride, more preferably tetraalkylammonium trichloride, even more preferably tetraethylammonium trichloride.

In some preferred embodiments this process also includes further steps, namely the steps of:
Ia) providing an alkanol having at least one, more than one, or all of the carbon atoms of its main chain replaced by ¹³C-atom(s),
IIa) converting the alkanol to a corresponding bromide,
IIIa) converting the bromide to a Wittig salt,
IVa1) coupling the Wittig salt with an aldehyde to form an alkene having at least one alpha,beta-ethylenically unsaturated double bond in its main chain, wherein the aldehyde optionally has one, more than one, or all of the carbon atoms of its main chain replaced with ¹³C-atom(s),
   or
IVa2) coupling the Wittig salt with a dialdehyde to form an alkene having at least two alpha,beta-ethylenically unsaturated double bonds in its main chain, wherein the dialdehyde optionally has one, more than one, or all of the carbon atoms
   of its main chain replaced with ¹³C-atom(s),
   or
Ib) providing an alkanol or alkenol, particularly alkenol, having at least one, more than one, or all of the carbon atoms of its main chain replaced by ¹³C-atom(s),
IIb) converting the alkanol or alkenol, particularly alkenol, to a corresponding halide, particularly a bromide,
IIIb) converting the halide to a Grignard reagent,
IVb1) coupling the Grignard with a tosylate species, particularly made from alcohol, via a carbon-carbon cross coupling reaction catalysed with copper, nickel or palladium complexes, particularly Li₂CuCl₄, to form an alkene having at least one alpha,beta-ethylenically unsaturated double bond in its main chain, wherein the alcohol species optionally has one, more than one, or all of the carbon atoms of its main chain replaced with ¹³C-atom(s),
   or
IVb2) coupling the Grignard with a divalent tosylate species made from diol, via a carbon-carbon cross coupling reaction to form an alkene having at least two alpha,beta-ethylenically unsaturated double bonds in its main chain, wherein the diol species optionally has one, more than one, or all of the carbon atoms of its main chain replaced with ¹³C-atom(s).

In some other preferred embodiments of the process according to the present invention, the employed alkene is a linear alkene or the alkenes are linear alkenes, respectively.

In one preferred variation of the present invention, in particular in order to prepare ¹³C-labelled chlorinated paraffins having an even number of chlorine atoms, the process of the present follows schemes 1a) or 1b), respectively (schemes 1a) and 1b) only differing from each other in that in scheme 1b) the aldehyde (4) also has a ¹³C-labelled carbon atom):
1a) 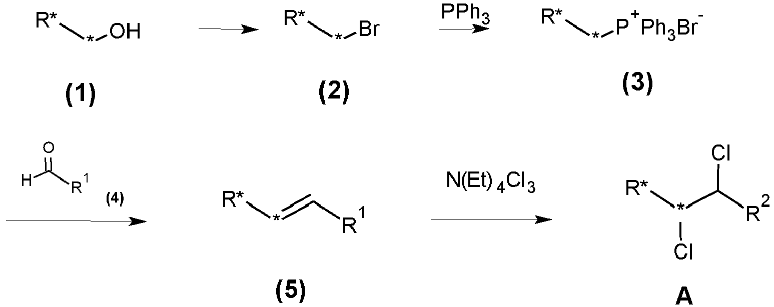
1b)

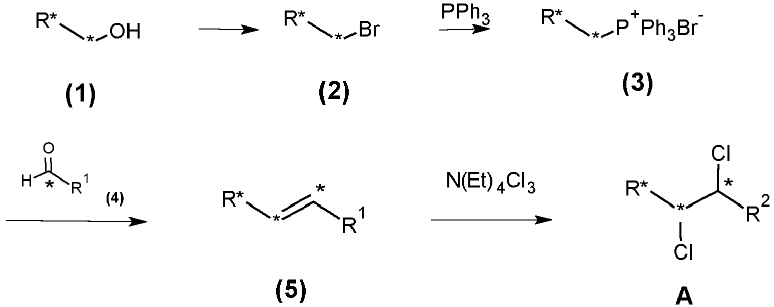

In another preferred variation of the present invention, in particular in order to prepare ¹³C-labelled chlorinated paraffins having an even number of chlorine atoms, the process of the present follows schemes 2a) or 2b), respectively (schemes 2a) and 2b) only differing from each other in that in scheme 2b) the aldehyde (6) also has a ¹³C-labelled carbon atom):
2a) 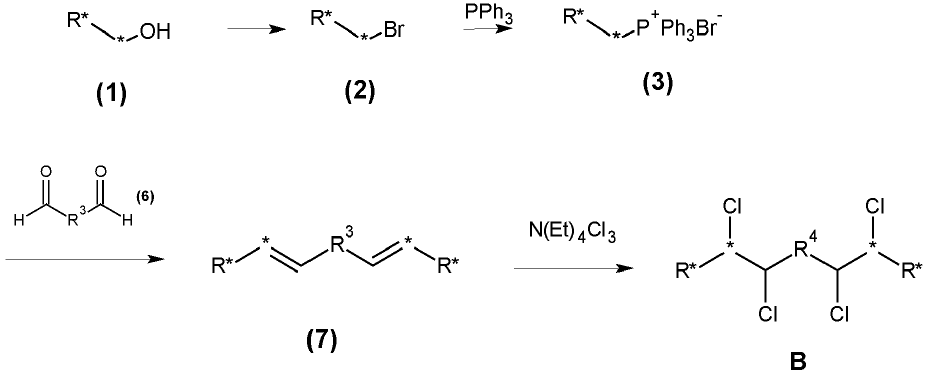
2b)

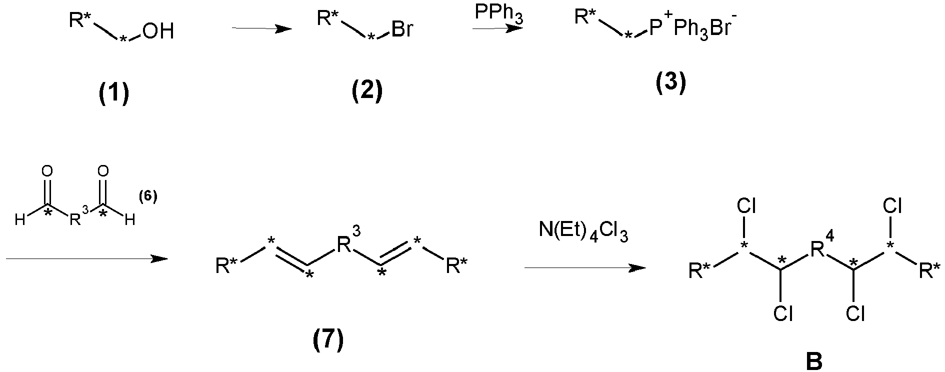

In yet another preferred variation of the present invention, in particular in order to prepare ¹³C-labelled chlorinated paraffins having an even number of chlorine atoms, the process of the present follows scheme 3:

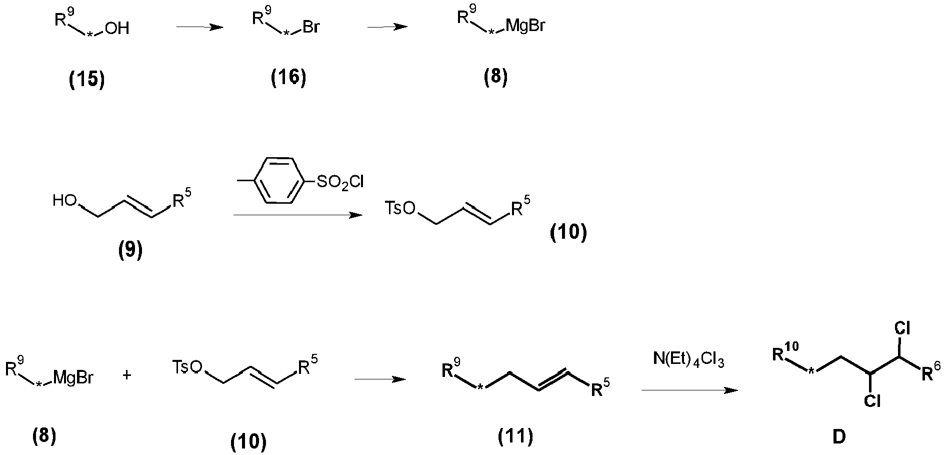

In still another preferred variation of the present invention, in particular in order to prepare ¹³C-labelled chlorinated paraffins having an even number of chlorine atoms, the process of the present follows scheme 4:

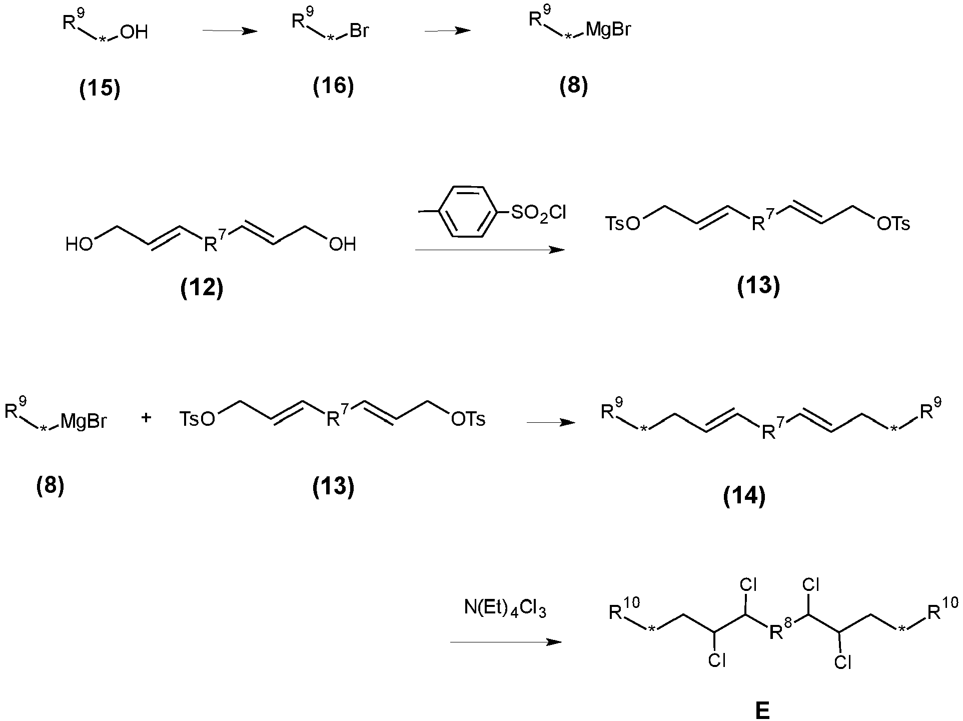

In all these variations the variables and residues are as follows:
R^{∗} = ¹³Cₐ-n-alkyl (straight chain alkyl), preferably of 0 to 4 carbon atoms, wherein a is the number of ¹³C in the alkyl chain and is from 1 up to the carbon number in the alkyl chain, preferably from 1 to 4;
^{∗} = ¹³C;
Ph = phenyl; Et = ethyl;
R¹ = ¹³C_{b}-alkylene-residue, preferably of 2-28 carbon atoms, wherein b is the number of ¹³C carbons in the alkylene residue and is from 1 up to the carbon number in the alkene chain, preferably from 1 to 4, optionally including one or an odd number of chlorine atoms with the proviso that no chlorine is on the terminal end of the residue, and wherein the number of double bonds in the alkylene residue is a minimum of one and up to the theoretically possible number of accumulated double bonds, particularly 1 to 9, and the double bonds are preferably not accumulated or conjugated;
R²= ¹³C_{b}-chloro-alkyl-residue, preferably of 2-28 carbon atoms, wherein b is the number of ¹³C carbons in alkyl residue, particularly of the formula ¹³C_{b}-CₙH_{2n+1-y}Cl_{y}, n is from 2 to 28 and y is the number of chlorine atoms and is from 2 to 28, but preferably 2 to 27;
R³ = ¹³C_{d}-alkylene-residue, preferably of 0 to 26 or of 1-26, carbon atoms, wherein d is the number of ¹³C carbons in alkylene residue and is from 1 up to the carbon number in the alkylene chain, preferably from 1 to 4, optionally including one or an odd number of chlorine atoms with the proviso that no chlorine is on the terminal end of the residue, and wherein the number of double bonds in the alkylene residue is a minimum of one and up to the theoretically possible number of accumulated double bonds, and the double bonds are preferably not accumulated or conjugated;
R⁴= ¹³C_{d}-chloro-alkyl-residue, preferably of 0 to 26 or of 1-26, carbon atoms, wherein d is the number of ¹³C carbons in alkyl residue, particularly of the formula ¹³C_{d}-CₙH_{2n-y}Cl_{y}, n is from 0 to 26 and y is the number of chlorine atoms and is from 0 to 26, but preferably 1 to 25;
R⁵ = ¹³Cₑ-alkylene-residue, preferably of 0 to 26 or of 1-26, carbon atoms, wherein e is the number of ¹³C in the alkyl or alkene chain and is from 1 up to the carbon number in the alkyl or alkene chain, preferably from 1 to 4, optionally including one or an odd number of chlorine atoms with the proviso that no chlorine is on the terminal end of the residue, and wherein the number of double bonds in the alkylene residue is a minimum of one and up to the theoretically possible number of accumulated double bonds, and the double bonds are preferably not accumulated or conjugated; R⁶=¹³Cₑ-chloro-alkyl-residue, preferably of 0 to 26 or of 1-26, of carbon atoms, wherein e is the number of ¹³C carbons in alkyl residue, particularly of the formula ¹³Cₑ-CₙH_{2n+1-y}Cl_{y}, n is from 1 to 26 and y is the number of chlorine atoms and is from 2 to 26, but preferably 2 to 25;
R⁷ = ¹³C_{f}-alkylene-residue, preferably of 0-22 carbon atoms, f is the number of ¹³C in the alkene chain and is from 1 up to the carbon number in the alkene chain, preferably from 1 to 4, optionally including one or an odd number of chlorine atoms, and wherein the number of double bonds in the alkylene residue is a minimum of one and up to the theoretically possible number of accumulated double bonds, and the double bonds are preferably not accumulated or conjugated;
R⁸= ¹³C_{f}-chloro-Cₙ-alkyl-residue, preferably of 0 to 22 carbon atoms, particularly of formula ¹³C_{f}-CₙH_{2n-y}Cl_{y}, n is 0 to 22 and y is the number of chlorine atoms and is from 0 to 22, but preferably 0 to 21;
R⁹= ¹³C_{g}-alkyl or alkene-residue, preferably of 0-4 carbon atoms if alkyl and of 3 or 4 carbon atoms if alkylene, wherein g is the number of ¹³C in the alkyl or alkene chain and is from 1 up to the carbon number in the alkyl or alkene chain, preferably from 1 to 4, and can contain 1 double bond;
R¹⁰ = ¹³C_{g}-(chloro)-alkyl, preferably of 0 to 4 carbon atoms, with 0, 1 or 2 chlorine atoms and is the same as R⁹ if R⁹ =¹³C_{g}-alkyl-residue; in some preferred embodiments, R⁹ = R¹⁰ = ¹³C_{g}-alkyl residue;
a, b, c, d, e, f, g are, independently from one another, an integer of from 1 up to the number of carbon atoms in the respective alkyl or alkylene residue; in some preferred embodiments they each are from 1 to 4.

It should be noted here, that in the present invention ^{∗} = ¹³C represents the carbon that is at the indicated position in the formula including the necessary hydrogen atoms (i.e. 0 to 3 hydrogen atoms) needed to satisfy the valencies of the carbon atom being at the indicated position (as is commonly done in chemical formulae).

It should also be noted that in the denotation of the present invention ¹³C_{index} means the indexed number of carbon atoms in the following given formula is replaced with ¹³C carbon atoms, it does not mean that the indexed number of carbons is added. For example, ¹³C₃-C₆H₁₂ would mean that three of the six carbon atoms are replaced with ¹³C isotopes. The exact position of the ¹³C-isotpoes in the carbon chain may be indicated by numerals preceding the ¹³C_{index}-term, for example 2,3,5-¹³C₃-C₆H₁₂ would mean that three of the six carbon atoms are replaced with ¹³C isotopes and that these are at chain positions 2, 3 and 5. It should also be understood, that the term ¹³C_{index} does not necessarily have to be in front of the remaining formula, in some instances it is better in the middle of the formula or at the end, in order to more clearly illustrate the structure and/or to increase intelligibility.

Compounds (1) are ¹³C-labelled alcohols, which are commercially available.

In preferred embodiments, R¹ has the same chain length as R².

In preferred embodiments, R³ has the same chain length as R⁴.

In preferred embodiments, R⁵ has the same chain length as R⁶.

In preferred embodiments, R⁷ has the same chain length as R⁸.

In preferred embodiments, R⁹ has the same chain length as R¹⁰.

It should also be noted that the process according to scheme 4 is employed with the proviso that it is only used to prepare ¹³C-labelled chlorinated paraffins in which the entire chain length is from 10 to 40 carbon atoms.

The unsaturated aldehydes **(4)** and **(6),** unsaturated alcohols **(9)** and **(12),** with or without chlorines in the carbon chain, can be prepared by methods known in the art. For example, alcohols can be oxidised according to the Swern reaction and then reacted with a Wittig salt to provide aldehydes having longer chains than the starting alcohol. However, there is no particular limitation on how the aldehydes (4) and (6) or the alcohols (9) and (12) may be prepared.

In some preferred embodiments of the present invention the aldehydes (4), (6) or alcohols (9) and (12) are prepared from unsaturated fatty acids. For example, this can be done by A) first providing an unsaturated fatty acid or a mixture of unsaturated fatty acids and then either Ca) converting the unsaturated fatty acid or unsaturated fatty acids to a corresponding alkenol or mixture of alkenols or Da) converting the unsaturated fatty acid or unsaturated fatty acids to a corresponding alkenal or mixture of alkenals. These embodiments are especially useful for the synthesis of medium and long chain CP (MCCP and LCCP).

In some preferred embodiments of the present invention the alkenes in step V) of the process of the present invention are prepared from unsaturated fatty acids, via the following steps:
A) providing an unsaturated fatty acid or a mixture of unsaturated fatty acids,
Ca) converting the unsaturated fatty acid or unsaturated fatty acids to a corresponding alkenol or mixture of alkenols, and
Cb1) reducing the alkenol or mixture of alkenols to the corresponding alkene or mixture of alkenes, or
Cc1) replacing the alcohol group or groups with a leaving group or groups, preferably tosylate,
Cc2) coupling an alkyl to the former alcohol carbon, thereby eliminating the leaving group and forming an alkene or mixture of alkenes; or
Da) converting the unsaturated fatty acid or unsaturated fatty acids to a corresponding alkenal or mixture of alkenals, and
Db1) coupling the alkenal or mixture of alkenals with Wittig salts to form the corresponding alkene or mixture of alkenes.

Another further embodiment of the present invention is a process for preparing ¹³C-labelled or unlabelled chlorinated paraffins, preferably having neither geminal nor terminal chlorine atoms in their structure, by converting a ¹³C-labelled or unlabelled unsaturated fatty acid or a mixture of ¹³C-labelled or unlabelled unsaturated fatty acids to a ¹³C-labelled or unlabelled chlorinated paraffins or a mixture of ¹³C-labelled or unlabelled chlorinated paraffins.

In a preferred embodiment of the present invention, this process comprises the steps of
A) providing a ¹³C-labelled or unlabelled unsaturated fatty acid or a mixture of ¹³C-labelled or unlabelled unsaturated fatty acids,
Ba) chlorinating the unsaturated fatty acid or unsaturated fatty acids and
Bb) decarboxylating of the resulting chlorinated acid or acids, or
Ca) converting the unsaturated fatty acid or unsaturated fatty acids to a corresponding alkenol or mixture of alkenols, and
Cb1) reducing the alkenol or mixture of alkenols to the corresponding alkene or mixture of alkenes, and
Cb2) chlorinating the resulting alkene or mixture of alkenes; or
Cc1) replacing the alcohol group or groups with a leaving group or groups, preferably tosylate,
Cc2) coupling an alkyl to the former alcohol carbon, thereby eliminating the leaving group and forming an alkene or mixture of alkenes, and
Cc3) chlorinating the resulting alkene or mixture of alkenes; or
Cd) chlorinating the alkenol or mixture of alkenols; or
Da) converting the unsaturated fatty acid or unsaturated fatty acids to a corresponding alkenal or mixture of alkenals, and
Db1) coupling the alkenal or mixture of alkenals with Wittig salts to form the corresponding alkene or mixture of alkenes,
Db1a) optionally using Wittig salts with at least one ¹³C-labelled carbon atom in the coupling group, and
Db2) chlorinating the resulting alkene or mixture of alkenes.

These processes can be illustrated as in the following schemes 5 and 6, wherein, as an example for illustration purposes only, alpha-linolenic acid is used (the process is of course not limited to linolenic acid):

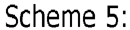

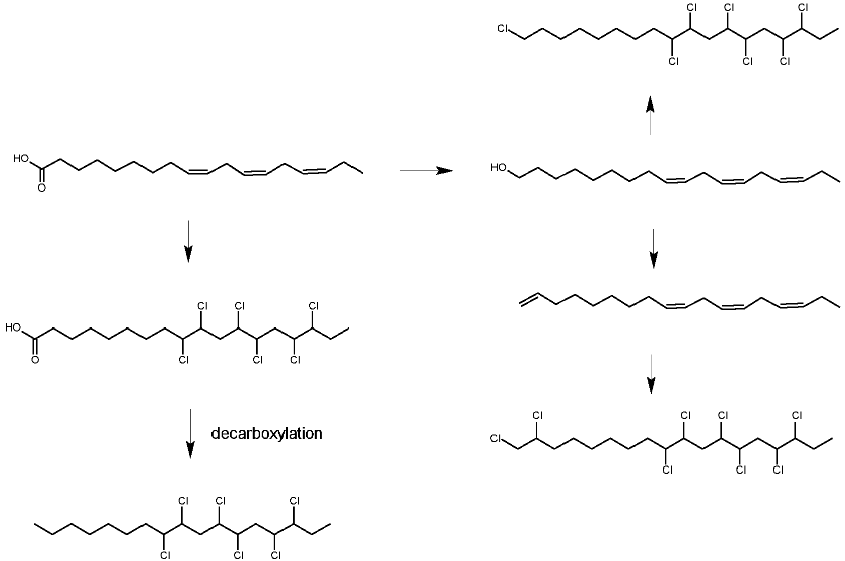

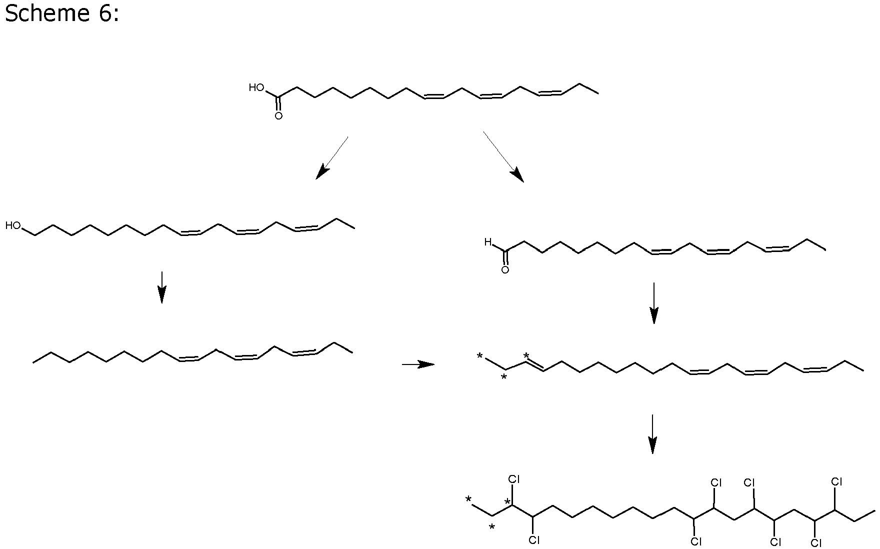

In preferred embodiments the fatty acids to be employed in this process are selected from C11-C24 unsaturated fatty acids, preferably C11 to C24 polyunsaturated fatty acids, more preferably even more preferably selected from, but not limited to, the following examples as well as mixtures thereof or, possibly, mixtures with other fatty acids: 9Z,12Z-Octadecadienoic acid (Linoleic acid, C18:2);
9Z,12Z,15Z-octadecatrienoic acid (alpha-linolenic acid, C18:3);
6Z,9Z,12Z-octadecatrienoic acid (gamma-linolenic acid, C18:3);
6Z,9Z,12Z,15Z-Octadecatetrawnoic acid (Stearidonic acid, C18:4);
10Z,13Z-Nonadecadienoic acid (C19:2);
5Z,8Z,11Z-Icosatrienoic acid (Mead acid, C20:3);
8Z,11Z,14Z-Eicosatrienoic acid (Homogamma linolenic acid, C20:3);
11Z,14Z,17Z-Eicosatrienoic acid (C20:3);
5Z,8Z,11Z,14Z-Eicosatetraenoic acid (Arachidonic acid, C20:4);
5Z,8Z,11Z,14Z,17Z-Eicosapentaenoic acid (EPA, C20:5).

Still even more preferably in some embodiments the fatty acids are selected from the group consisting of 9Z,12Z-Octadecadienoic acid (Linoleic acid, C18:2), 9Z,12Z,15Z-octadecatrienoic acid (alpha-linolenic acid, C18:3), 6Z,9Z,12Z-octadecatrienoic acid (gamma-linolenic acid, C18:3), 6Z,9Z,12Z,15Z-Octadecatetrawnoic acid (Stearidonic acid, C18:4), 10Z,13Z-Nonadecadienoic acid (C19:2), 5Z,8Z,11Z-Icosatrienoic acid (Mead acid, C20:3), 8Z,11Z,14Z-Eicosatrienoic acid (Homogamma linolenic acid, C20:3), 11Z,14Z,17Z-Eicosatrienoic acid (C20:3), 5Z,8Z,11Z,14Z-Eicosatetraenoic acid (Arachidonic acid, C20:4), 5Z,8Z,11Z,14Z,17Z-Eicosapentaenoic acid (EPA, C20:5) and mixtures thereof.

In another embodiment, the present invention is directed to ¹³C-labelled chlorinated paraffins having neither geminal nor terminal chlorine atoms in their structure, having
- at least two chlorine atoms in their main chain, and
- at least one, particularly from two to eight, carbon atoms of the main chain replaced by ¹³C-atoms.

Preferably these ¹³C-labelled chlorinated paraffins are represented by the general formula ¹³Cₘ-CₚH₂ₚ₊₂₋ₓClₓ, wherein the variables have the following meaning:
m is an integer of from 1 to p, particularly 2 to 8;
p is an integer of from 2 to 38, preferably from 8 to 38, particularly 8 to 30;
x is from 2 to p-2, preferably 4 to p-2, preferably, in the case of SCCP 4 to 6, particularly 4 or 6, in the case of MCCP 6 to 8, particularly 6 or 8, in the case of LCCP 8 to 10, particularly 8 or 10, one especially preferred embodiment is 4 or 6.

Optionally, and in some embodiments of the present invention preferably, these ¹³C-labelled chlorinated paraffins are prepared according to a process according to the present invention as outlined above.

In yet another embodiment, the present invention is directed to ¹³C-labelled chlorinated paraffins having neither geminal nor terminal chlorine atoms in their structure, optionally and in some embodiments preferred, as described above, which can be (are) represented by
- either general formula A 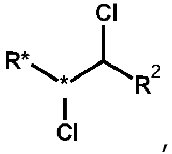
- or general formula B 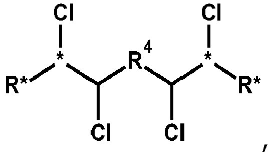
- or general formula D 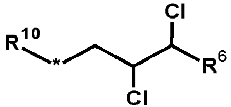
- or general formula E 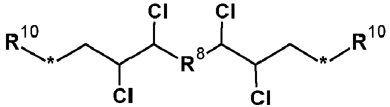 wherein the variables and residues have the meaning defined above.

In still another embodiment, the present invention is directed to ¹³C-labelled chlorinated paraffins having neither geminal nor terminal chlorine atoms in their structure, optionally and in some embodiments preferred, as described above, which can be (are) represented by
- general formula A 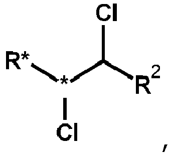 wherein ^{∗} = ¹³C and R^{∗} are as defined above, or are selected from the group consisting of
   i) 6,7,8-¹³C₃-2,3,5,6-tetrachlorooctane or 2,3,5,6-tetrachlorooctane-6,7,8-¹³C₃ of formula 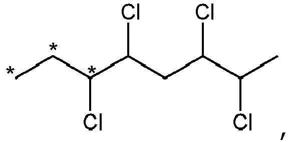
   ii) 1,2,9,10-¹³C₄-2,3,8,9-tetrachlorodecane 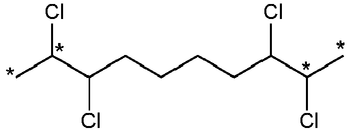
   iii) 9,10,11-¹³C₃-2,3,5,6,8,9-hexachloroundecane or 2,3,5,6,8,9-hexachloroundecane-9,10,11-¹³C₃ of formula 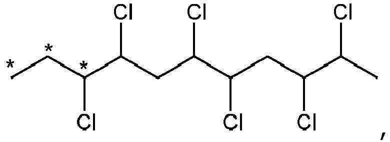
   iv) 11,12,13-¹³C₃-3,4,6,7,10,11-hexachlorotridecane or 3,4,6,7,10,11-hexachlorotridecane-11,12,13-¹³C₃ of formula 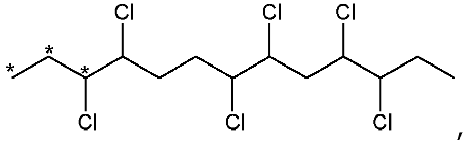
   v) 1,2,9,10-¹³C₄-2,3,5,6,8,9-hexachlorodecane-1,2,9,10-¹³C₄ or 2,3,5,6,8,9-hexachlorodecane-1,2,9,10-¹³C₄ of formula 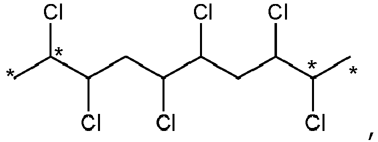
   vi) 6,7,8-¹³C₃-2,3,5,6,8,9,11,12-octachlorotridecane or 2,3,5,6,8,9,11,12-octachlorotridecane-6,7,8-¹³C₃ of formula 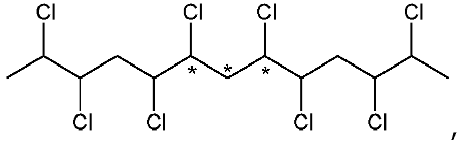
   vii) 10,11,12-¹³C₃-2,3,5,6,9,10-hexachlorododecane 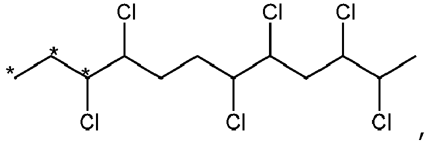 or
   viii) 10,11,12-¹³C₃-2,3,5,6,8,9-hexachlorododecane 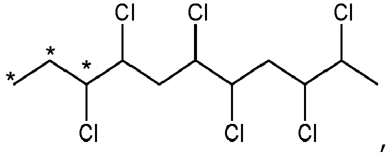
   ix) 1,2,3-¹³C₃-3,4,7,8,11,12-hexachlorotetradecane 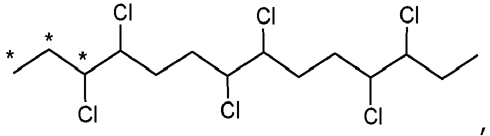
   x) 13,14,15-¹³C₃-3,4,7,8,12,13-hexachloropentadecane 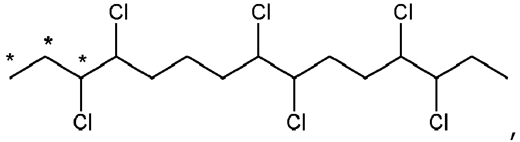
   xi) 1,2,3-¹³C₃-3,4,8,9,13,14-hexachlorohexadecane 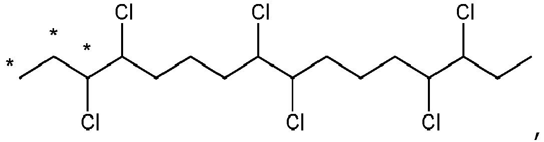
   xii) 1,2,3-¹³C₃-3,4,12,13,15,16,18,19-octachloro-n-heneicosane, 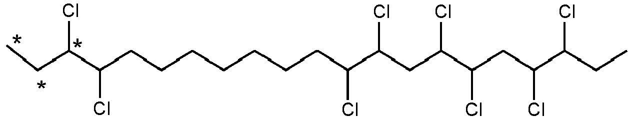 and mixtures thereof,
   wherein ^{∗} is as defined above.

In one embodiment of the present invention are those of i), iii), iv) and v) preferred.

In still a further embodiment, the present invention is directed to mixtures of chlorinated paraffins comprising ¹³C-labelled chlorinated paraffins having neither geminal nor terminal chlorine atoms in their structure according to the present invention and as described above or prepared according to the process of the present invention as described above.

In yet another further embodiment, the present invention is directed to the use of ¹³C-labelled chlorinated paraffins having neither geminal nor terminal chlorine atoms in their structure according to the present invention and as described above or prepared according to the process of the present invention as described above as internal standards for analytical processes, in particular for the quantitative analysis of very short-chain chlorinated paraffins (vSCCPs, <C10), short-chain chlorinated paraffins (SCCPs, C10-C13); medium-chain chlorinated paraffins (MCCPs, C14-C17) and long-chain chlorinated paraffins (LCCPs, C18-C30).

Still another embodiment of the present invention is the use of ¹³C-labelled chlorinated paraffins having neither geminal nor terminal chlorine atoms in their structure according to the present invention and as described above or prepared according to the process of the present invention as described above as reference materials for metabolism and toxicity analysis of chlorinated paraffins.

One further embodiment of the present invention is directed to chlorinated paraffins selected from the group consisting of 9,10,11-¹³C₃-1,2,4,5,8,9-hexachloroundecane, 10,11,12-¹³C₃-1,2,5,6,9,10-hexachlorododecane, 11,12,13-¹³C₃-1,2,6,7,10,11-hexachlorotridecane, 12,13,14-¹³C₃-1,2,7,8,11,12-hexachlorotetradecane, 13,14,15-¹³C₃-1,2,7,8,12,13-hexachloropentadecane, 14,15,16-¹³C₃-1,2,8,9,13,14-hexachlorohexadecane, 1,2,9,10-¹³C₄-2,3,8,9-tetrachlorodecane, 8,9,10-¹³C₃-1,2,4,5,7,8-hexachlorodecane, 1,2,3-¹³C₃-3,4,12,13,15,16,18,19-octachloro-n-heneicosane and mixtures thereof, particularly 1,2,9,10-¹³C₄-2,3,8,9-tetrachlorodecane or 8,9,10-¹³C₃-1,2,4,5,7,8-hexachlorodecane.

Not the least, the present invention in other embodiments is directed to internal standards for analytical processes, in particular for the quantitative analysis of vSCCPs, SCCPs, MCCPs, LCCPs, comprising or consisting of
- one or more of the ¹³C-labelled chlorinated paraffins having neither geminal nor terminal chlorine atoms in their structure according to the present invention and as described above or prepared according to the process of the present invention as described above,
- one or more of the chlorinated paraffins selected from the group consisting of 9,10,11-¹³C₃-1,2,4,5,8,9-hexachloroundecane, 10,11,12-¹³C₃-1,2,5,6,9,10-hexachlorododecane, 11,12,13-¹³C₃-1,2,6,7,10,11-hexachlorotridecane, 12,13,14-¹³C₃-1,2,7,8,11,12-hexachlorotetradecane, 13,14,15-¹³C₃-1,2,7,8,12,13-hexachloropentadecane, 14,15,16-¹³C₃-1,2,8,9,13,14-hexachlorohexadecane, 1,2,9,10-¹³C₄-2,3,8,9-tetrachlorodecane, 8,9,10-¹³C₃-1,2,4,5,7,8-hexachlorodecane, 1,2,3-¹³C₃-3,4,12,13,15,16,18,19-octachloro-n-heneicosane and mixtures thereof, particularly 1,2,9,10-¹³C₄-2,3,8,9-tetrachlorodecane or 8,9,10-¹³C₃-1,2,4,5,7,8-hexachlorodecane,
- or a mixture of one or more of the chlorinated paraffins selected from the group consisting of 9,10,11-¹³C₃-1,2,4,5,8,9-hexachloroundecane, 10,11,12-¹³C₃-1,2,5,6,9,10-hexachlorododecane, 11,12,13-¹³C₃-1,2,6,7,10,11-hexachlorotridecane, 12,13,14-¹³C₃-1,2,7,8,11,12-hexachlorotetradecane, 13,14,15-¹³C₃-1,2,7,8,12,13-hexachloropentadecane, 14,15,16-¹³C₃-1,2,8,9,13,14-hexachlorohexadecane, 1,2,9,10-¹³C₄-2,3,8,9-tetrachlorodecane, ¹³C₃-1,2,4,5,7,8-hexachlorodecane, 1,2,3-¹³C₃-3,4,12,13,15,16,18,19-octachloro-n-heneicosane and mixtures thereof, particularly 1,2,9,10-¹³C₄-2,3,8,9-tetrachlorodecane or 8,9,10-¹³C₃-1,2,4,5,7,8-hexachlorodecane, and one or more other of the ¹³C-labelled chlorinated paraffins having neither geminal nor terminal chlorine atoms in their structure according to the present invention and as described above or prepared according to the process of the present invention as described above.

In preferred variations of this embodiment, these internal standards for analytical processes comprise or consist of single chain CP mixtures of ¹³Cₘ-CₚH₂ₚ₊₂₋ₓClₓ, with m being an integer of from 1 to p, particularly 2 to 8; p being an integer ≥ 8, particularly 8 to 30; x = 2 to p-1, preferably 2 to 10 or 2 to 7 if p=8, or x= 2 to p-2, preferably 4 to p-2; and wherein the ¹³C-labelled single-chain mixtures have chlorine contents of 45-75 weight%, calculated from the analysis on NMR and chlorine titration.

It is to be understood that deuterium atoms and ¹³C atoms occur naturally. Accordingly, any reference to replacing (or the like formulations) in the context of the present invention when relating to the atoms of the glycerol moiety or the alkanol moiety are to be understood as intentional replacements and do not take into account possible naturally occurring deuterium or ¹³C-atoms.

The present invention offers a number of advantages over the prior art, a few of which are:
- The process of the present invention for preparing ¹³C labelled chlorinated paraffins, particularly ones that have neither geminal nor terminal chlorine atoms is able to provide these CP with high reliability, good yield and high purity.
- The novel ¹³C labelled chlorinated paraffins, particularly ones that have neither geminal nor terminal chlorine atoms, are of high purity and have very well-defined chlorine content, which also makes quantification of CPs accurate, if they are used as standards, particularly internal standards.
- The novel ¹³C labelled chlorinated paraffins, particularly ones that have neither geminal nor terminal chlorine atoms, are very well suitable as reference materials, because they have a chlorine pattern which is similar to the CP analytes found in the technical mixtures or in the environment.
- The reliable preparation of the novel ¹³C labelled chlorinated paraffins, particularly ones that have neither geminal nor terminal chlorine atoms, ensures that they can be used as Certified Reference Materials (CRMS) for CPs analysis.

The invention is now described in more detail with reference to the following non-limiting examples. The following exemplary, non-limiting examples are provided to further describe the embodiments presented herein. Those having ordinary skill in the art will appreciate that variation of these examples are possible within the scope of the invention.

### Examples:

### General Synthesis procedures:

### Synthesis of ¹³C-labelled 1-bromoalkane and 1-bromoalkene (2 and 16). General Procedure A:

¹³C-labelled-n-alcohol **(1 or 15**) was converted to the bromides (**2 or 16**) by conventional methods, either by reaction with HBr-H₂SO₄ or with PBr₃; or in some cases the alcohol was transferred to tosylate first which was then reacted with LiBr to form the bromides.

A typical procedure of the reaction with PBr₃ which was followed is follows: ¹³C-labelled-n-alcohol (**1**) (3 mmol) and pyridine (75 mg) were mixed in pentane (5 ml) and cooled to -40°C. A solution of phosphorus tribromide (32 mg, 1.2 mmol) in pentane (2 ml) was added dropwise to the stirred solution. The mixture was stirred at the cooled temperature for 1 h, and the products were distilled off. The obtained solution of the product was diluted with pentane (10 ml), washed with 5% HCI, 10% HNO₃, water, 5% KHCO₃. The organic phase was dried over MgSO₄, filtered, concentrated. The residue was distilled to give the pure bromides (**2**), yield 75-80%.

### Synthesis of the Wittig salt (3). General procedure B:

The bromide (**2**) (10.0 mmol) and PPh₃ (10.0 mmol) were mixed in dry acetonitrile (25 ml), the mixture was heated to reflux for 72 to 48 hours. The solvent was evaporated and the crude product was purified by column chromatography (silica; eluent EtOAc : MeOH from 90:10, 80:20 to 75:25). Obtained product was dried in vacuo, yield 84%, of white solid **3.**

### Synthesis of ¹³C-labelled alkenes (5 and 7). General procedure C:

Wittig salt (**3**) (15.6 mmol) wax mixed in dry THF (50 ml) under argon and cooled to -78°C, n-butyllithium (2.5 M, 6.85 ml) was added dropwise. The reaction mixture was then warmed to -40°C to -10°C and kept at this temperature for 1.5 hours, before it was cooled to -78°C again and the aldehyde (**4**) (15.9 mmol) was added slowly. The reaction mixture was warmed to room temperature and stirred for 16 hours, saturated NH₄Cl solution (50 ml) was added to quench the reaction. The two phases were separated, water phase was extracted with diethyl ether and the combined organic phases were dried over MgSO₄, filtered and concentrated under reduced pressure. The crude product was purified by column chromatography (silica; eluent petroleum ether) and afforded the ¹³C-labelled alkene (**5**), yield 85-90%.

The ¹³C-labelled alkenes (**7**) were synthesized in the same way by using 2 equivalents of the Wittig salt (**3**) and 1 equivalent of the dialdehydes (**6**).

### Synthesis of ¹³C-labelled alkenes (11 and 14). General procedure D:

### a) The Grignard reagent 8

Magnesium (1.75 g, 72.0 mmol), dry diethyl ether (6 ml) and a crystal of iodide was added a few drops of the bromide (**2**) in dry diethyl ether until the reaction started (under argon). The solution of the bromide (**2**) (total 53.41 mmol) in diethyl ether (18 ml) was added dropwise at such rate as to maintain gentle reflux. After the addition was complete, the mixture was refluxed for 1 hour. The mixture was cooled and more diethyl ether (2.5 ml) added. The mixture (about 2 M) was used directly in the synthesis of **11 or 14.**

### b) The tosylates 10 and 13

p-toluenesulfonyl chloride (7.72 g; 40.5 mmol) in dry DCM (50 ml) was added via dropping funnel to a solution of the alcohol (**9**; 36.8 mmol) and triethylamine (11.3 ml; 80.9 mmol) in dry dichloromethane (200 ml) at 0°C in argon atmosphere. The reaction mixture was stirred at 0°C for 15 minutes, then at room temperature overnight (18 hours). The reaction was quenched with water (250 ml). Two phases were separated and the water phase was extracted with DCM (3×100 ml). The combined organic phases were washed with water, 10% NaHCO₃ and water (all 200 ml) and dried over MgSO₄. Crude product was purified by column chromatography (silica; eluent petroleum ether : DCM from 60:40, 50:50 to 25:75) to give the tosylate **10,** yield 80-85%

The ditosylate **13** was made in the same manner from the diol compound **12** (1 equivalent), triethylamine (8 equivalents) and p-toluenesulfonyl chloride (2,2 equivalents) in DCM. The crude product was purified by column chromatography (silica; eluent petroleum ether : DCM from 60:40, 50:50 to 25:75) to give the tosylate **13** as white solid. Recrystallisation in methanol or dichloromethane was also used, this gave a white crystalline product with yields from 34 - 55%.

### c) Cross coupling reaction, the alkenes 11 and 14

The solution of **8** in diethyl ether (about 2 M) under argon was cooled to -75°C and a solution of **10** (50 mmol) in THF (50 ml) and dilithium tetrachlorocuprate (10 mmol) (0.1M solution in THF, 100ml) was added. The temperature increased slowly to around about -30°C and was kept for 15 minutes. The mixture was cooled one more time to -70°C and more of the dilithium tetrachlorocuprate solution was added (0.2 ml, 0.02 mmol), the temperature was then increased slowly and the mixture was stirred at room temperature overnight. Silica (50 g) was added to the mixture and the solvents were evaporated. The dry residue was poured on top on a column filled with silica and eluated with heptane. This gave the pure alkene **11,** yield 50-60%.

The ¹³C-labelled alkene (**14**) was synthesized in the same way by using 2 equivalents of the Grignard reagent (**8**) and 1 equivalent of the di-tosylate (**13**).

### Synthesis of tetraethylammonium trichloride Et₄N(Cl)₃:

A solution of tetraethylammonium chloride (Et₄NCl) in DCM was saturated with Cl₂, the solvent was then removed in vacuo to afford a yellow solid which was stored under argon for later use in the chlorination reaction.

### Chlorination and Synthesis of ¹³C-labelled CP (A, B, D and E). General procedure X:

A solution of Et₄NCl₃ (1.5 - 2.0 equivalents for each C=C bond) in DCM was slowly added via syringe into the solution of the substrate in DCM (**5**, 0.3 M) at -78°C in argon atmosphere until the reaction mixture turned yellow. The mixture was stirred for 20 minutes at -78°C, more trichloride solution was added if the yellow colour disappeared.

After the reaction was quenched with pent-1-ene, the mixture was allowed to warm to room temperature and the Et₄NCl solid formed in the reaction dissolved. The solvent is removed in vacuo and the residue was treated with diethyl ether, which results in the precipitation of Et₄NCl again, the solid was filtered off, washed with EtzO and filtrate evaporated to dryness. The crude product was further purified by column chromatography. All ¹³C-labelled products were characterized by NMR and GC-MS.

### Synthesis examples:

The following ¹³C-labelled CP are listed as examples for further understanding of the present invention, but the content of this invention is not limited to these. ¹³C₃-labelled CP synthesized from ¹³C₃-1-propanol and straight-chain unsaturated aldehydes (minimum one double bond in the carbon chain) (Scheme 1) was taken as examples. The synthesis of 9,10,11-¹³C₃-2,3,5,6,8,9-Hexachlorododecane is described in detail as below:

### Example: Synthesis of 9,10,11-¹³C₃-2,3,5,6,8,9-Hexachlorododecane

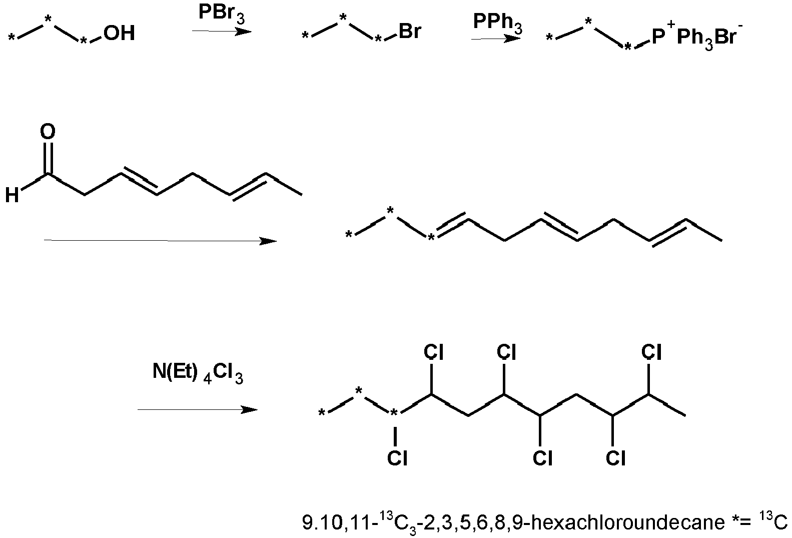

### ¹³C₃-1-bromopropane

¹³C₃-1-propanol (0,97 g, 15.4 mmol) and pyridine (375 mg, 4.75 mmol) were mixed in pentane (25 ml) and cooled to -40°C. A solution of phosphorus tribromide (1.68 g, 6.2 mmol) in pentane (10 ml) was added dropwise to the stirred solution. The mixture was stirred at the cooled temperature for 1 hour, and the products were distilled off. The obtained solution of the product was diluted with pentane (50 ml), washed with 5% HCl, 10% HNO₃, water, 5% KHCO₃. The organic phase was dried over MgSO₄, filtered, concentrated. The residue was distilled to give 1.52 g (12.1 mmol) ¹³C₃-1-bromopropane, light yellow oil, yield 78.6%.

### Triphenyl-¹³C₃-propyl phosphonium bromide, the Wittig salt

¹³C₃-1-bromopropane (1.26 g, 10.0 mmol) and PPh₃ (2.63 g, 10.0 mmol) were mixed in dry acetonitrile (25 ml), the mixture was heated to reflux for 72 hours. The solvent was evaporated and the crude product was purified by column chromatography (silica; eluent EtOAc : MeOH from 90:10, 80:20 to 75:25). Obtained product was dried in vacuo, yield 84%, 3,26 g (8.4 mmol) white solid powder.

### Synthesis of 9,10,11-¹³C₃-undeca-2,5,8-triene

The Wittig salt (3.0 g, 7.8 mmol) wax mixed in dry THF (25 ml) under argon and cooled to -78°C, n-butyllithium (2.5 M, 3.4 ml) was added dropwise. The reaction mixture was then warmed to -40°C to -10°C and kept at this temperature for 1.5 hours, before it was cooled to -78°C again and the aldehyde octa-3,6-dienal (1.0 g, 8.0 mmol) was added slowly. The reaction mixture was warmed to room temperature and stirred for 16 hours, saturated NH₄Cl solution (25 ml) was added to quench the reaction. The two phases were separated, water phase was extracted with diethyl ether and the combined organic phases were dried over MgSO₄, filtered and concentrated under reduced pressure. The crude product was purified by column chromatography (silica; eluent petroleum ether) and afforded the ¹³C-labelled alkene 9,10,11-¹³C₃-undeca,2,5,8-triene, 1.02g (6.7 mmol), yield 86%.

### Synthesis of tetraethylammonium trichloride Et₄N(Cl)₃

A solution of tetraethylammonium chloride (Et₄NCl) in DCM was saturated with Cl₂, the solvent was then removed in vacuo to afford a yellow solid which was stored under argon for later use in the chlorination reaction.

### 9,10,11-¹³C₃-2.3.5.6,8,9-Hexachlorododecane

A solution of Et₄NCl (9.26 g, 39.1 mmol, 6.0 equivalents) in DCM (50 ml) is slowly added via syringe into the solution of 9,10,11-¹³C₃-undeca,2,5,8-triene 1,0 g, 6.5 mmol in DCM (130 ml) at -78°C in argon atmosphere until the reaction mixture turned yellow. The mixture was stirred for 25 minutes at -78°C, then quenched with excess of pent-1-ene. Solvent was evaporated and the crude product purified by column chromatography (eluent petroleum ether°: diethyl ether = 90:10) and by recrystallization from n-hexane to give 1.23 g (3.25 mmol) of the target compound 9,10,11-¹³C₃-2,3,5,6,8,9-Hexachlorododecane, yield 50.0%, purity >99.0% analysed by GC and NMR.

### Other examples of ¹³C₃-labelled CP

Starting from ¹³C₃-propanol and an unsaturated aldehyde having 1-2 double bonds, ¹³C₃-CP having 4 or 6 chlorines (Cl₄ or Cl₆) can be synthesized in the same way as 9,10,11-¹³C₃-2,3,5,6,9,10-hexachlorododecane; The general structure of ¹³C₃-Cl₆-CP is as shown in the following formula. 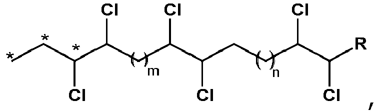 with m≥1, n≥0, R=n-alkyl, R can also be proton (H).

The following table 1 shows ¹³C₃-CP and ¹³C₄-CP without terminal and geminal chlorines, table 2 shows ¹³C₃-CP with one terminal chlorine that were all prepared with the process according to the present invention.

**Table 1. Examples of ¹³C₃ and ¹³C₄ Cl₄ or Cl₆-CP according to and prepared with the process according to the present invention**

| Structure | Name |
|---|---|
| 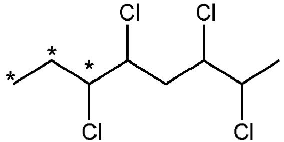 | 6,7.8-¹³C₃-2,3,5,6-tetrachlorooctane (¹³C₃-C₈Cl₄) |
| 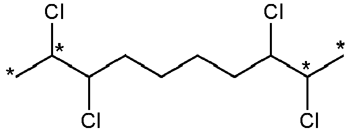 | 1,2,9,10-13C4-2,3,8,9-tetrachlorodecane (¹³C₄-C₁₀Cl₄) |
| 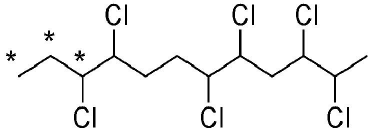 | 10,11,12-¹³C₃-2,3,5,6,9,10-hexachlorododecane (¹³C₃-C₁₂Cl₆) |
| 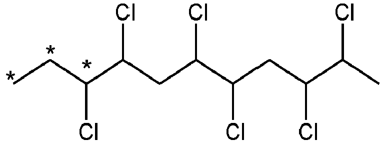 | 10,11,12-¹³C₃-2,3,5,6,8,9-hexachlorododecane (¹³C₃-C₁₂Cl₆) |
| 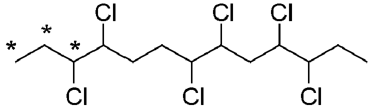 | 11,12,13-¹³C₃-3,4,6,7,10,11-hexachlorotridecane (¹³C₃-C₁₃Cl₆) |
| 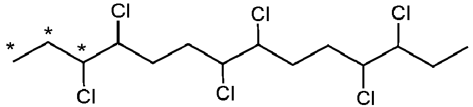 | 1,2,3-¹³C₃-3,4,7,8,11,12-hexachlorotetradecane (¹³C₃-C₁₄Cl₆) |
| 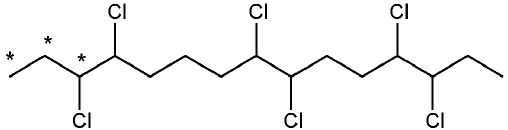 | 13,14,15-¹³C₃-3,4,7,8,12,13-hexachloropentadecane (¹³C₃-C₁₅Cl₆) |
| 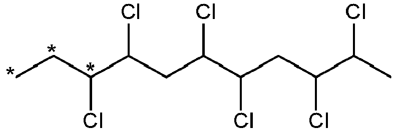 | 9,10,11-¹³C₃-2,3,5,6,8,9-hexachloroundecane (or 2,3,5,6,8,9-hexachloroundecane-9,10,11-¹³C₃) |
| 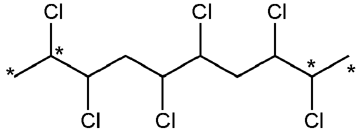 | 1,2,9,10-¹³C₄-2,3,5,6,8,9-hexachlorodecane-1,2,9,10-¹³C₄ (or 2,3,5,6,8,9-hexachlorodecane-1,2,9,10-¹³C₄) |
| 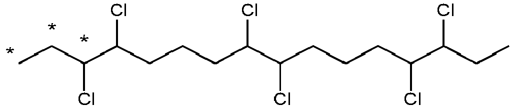 | 1,2,3-¹³C₃-3,4,8,9,13,14-hexachlorohexadecane (¹³C₃-C₁₆Cl₆) |

**Table 2 ¹³C₃-CPs with terminal chlorine prepared with the process according to the present invention**

| Structure | Name |
|---|---|
| 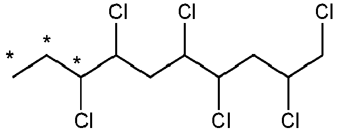 | 9,10,11-¹³C₃-1,2,4,5,7,8-hexachloroundecane |
| 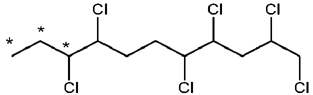 | 9,10,11-¹³C₃-1,2,4,5,8,9-hexachloroundecane |
| 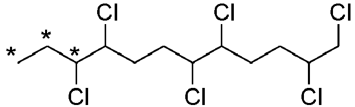 | 10,11,12-¹³C₃-1,2,5,6,9,10-hexachlorododecane |
| 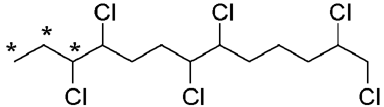 | 11,12,13-¹³C₃-1,2,6,7,10,11-hexachlorotridecane |
| 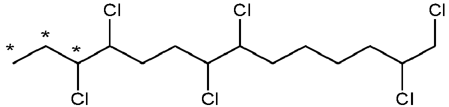 | 12,13,14-¹³C₃-1,2,7,8,11,12-hexachlorotetradecane |
| 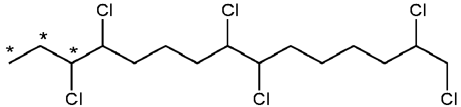 | 13,14,15-¹³C₃-1,2,7,8,12,13-hexachloropentadecane |
| 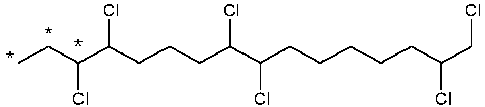 | 14,15,16-¹³C₃-1,2,8,9,13,14-hexachlorohexadecane |

## Claims

1. Process for preparing ¹³C-labelled chlorinated paraffins, preferably having neither geminal nor terminal chlorine atoms in their structure, comprising or consisting of the steps of:
V) providing an alkene
- having one or more alpha,beta-ethylenically unsaturated double bonds in its main chain,
- one or more carbon atoms in its main chain being replaced by ¹³C-atom(s),
- and optionally one chlorine atom or an odd number of chlorine atoms in its main chain, with the proviso that neither geminal nor terminal chlorines are present and none are present on an atom that is part of an alpha,beta-ethylenically unsaturated double bond,
or a mixture of such alkenes,
VI) reacting the alkene or mixture of alkenes with a trichloride, a tribromide or triiodide, preferably a trichloride, more preferably tetraalkylammonium trichloride, even more preferably tetraethylammonium trichloride.

2. Process according to claim 1, **characterized in that** it additionally includes the steps of
Ia) providing an alkanol having at least one, more than one, or all of the carbon atoms of its main chain replaced by ¹³C-atom(s),
IIa) converting the alkanol to a corresponding bromide,
IIIa) converting the bromide to a Wittig salt,
IVa1) coupling the Wittig salt with an aldehyde to form an alkene having at least one alpha,beta-ethylenically unsaturated double bond in its main chain, wherein the aldehyde optionally has one, more than one, or all of the carbon atoms of its main chain replaced with ¹³C-atom(s),
or
IVa2) coupling the Wittig salt with a dialdehyde to form an alkene having at least two alpha,beta-ethylenically unsaturated double bonds in its main chain, wherein the dialdehyde optionally has one, more than one, or all of the carbon atoms of its main chain replaced with ¹³C-atom(s),
or
Ib) providing an alkanol or alkenol, particularly alkenol, having at least one, more than one, or all of the carbon atoms of its main chain replaced by ¹³C-atom(s),
IIb) converting the alkanol or alkenol, particularly alkenol, to a corresponding halide, particularly a bromide,
IIIb) converting the halide to a Grignard reagent,
IVb1) coupling the Grignard with a tosylate species, particularly made from alcohol, via a carbon-carbon cross coupling reaction catalysed with copper, nickel or palladium complexes, particularly Li₂CuCl₄, to form an alkene having at least one alpha,beta-ethylenically unsaturated double bond in its main chain, wherein the alcohol species optionally has one, more than one, or all of the carbon atoms of its main chain replaced with ¹³C-atom(s),
or
IVb2) coupling the Grignard with a divalent tosylate species made from diol, via a carbon-carbon cross coupling reaction to form an alkene having at least two alpha,beta-ethylenically unsaturated double bonds in its main chain, wherein the diol species optionally has one, more than one, or all of the carbon atoms of its main chain replaced with ¹³C-atom(s).

3. Process according to claim 1 or claim 2, wherein the alkene is a linear alkene or the alkenes are linear alkenes, respectively.

4. Process according to claim 3, **characterized in that**, in particular in order to prepare ¹³C-labelled paraffins having an even number of chlorine atoms, it follows
- either scheme 1a): 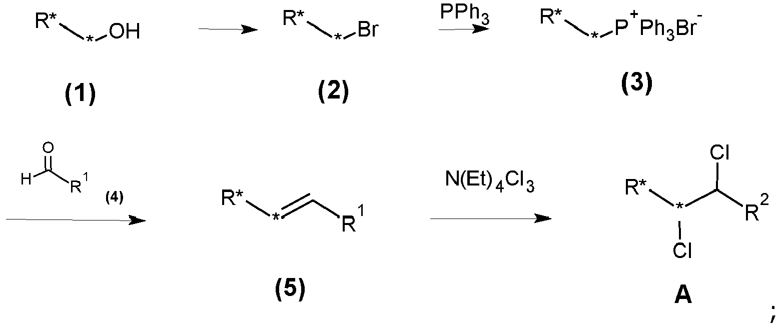
- or scheme 1b): 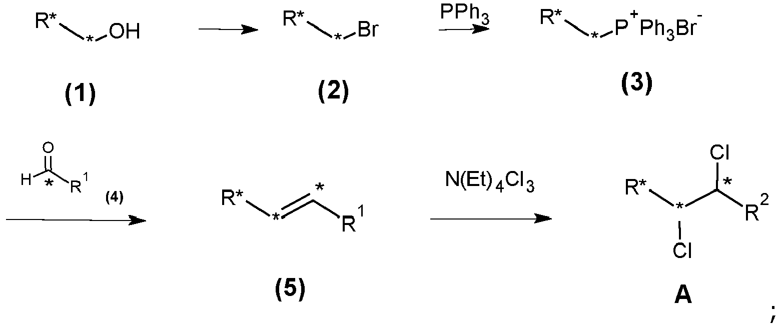
- or scheme 2a): 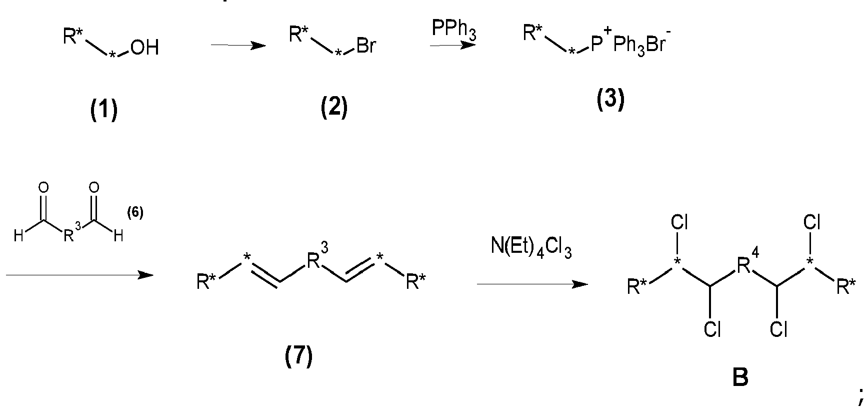
- or scheme 2b): 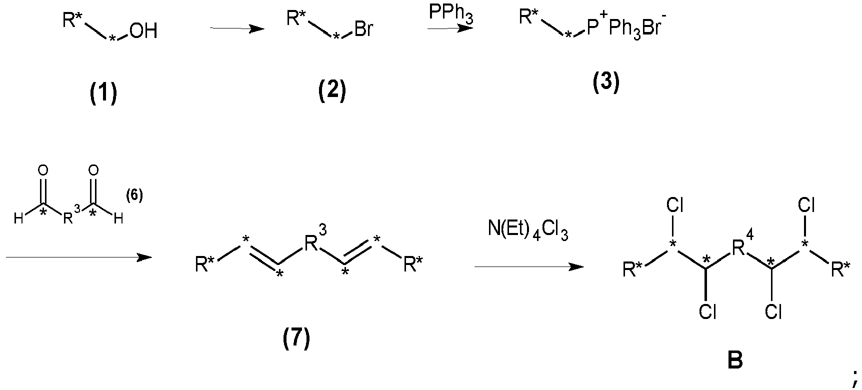
- or scheme 3: 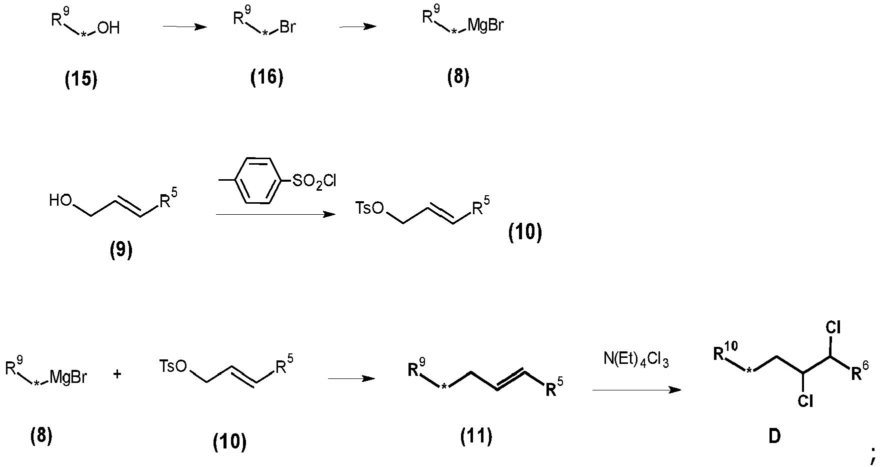
- or scheme 4: 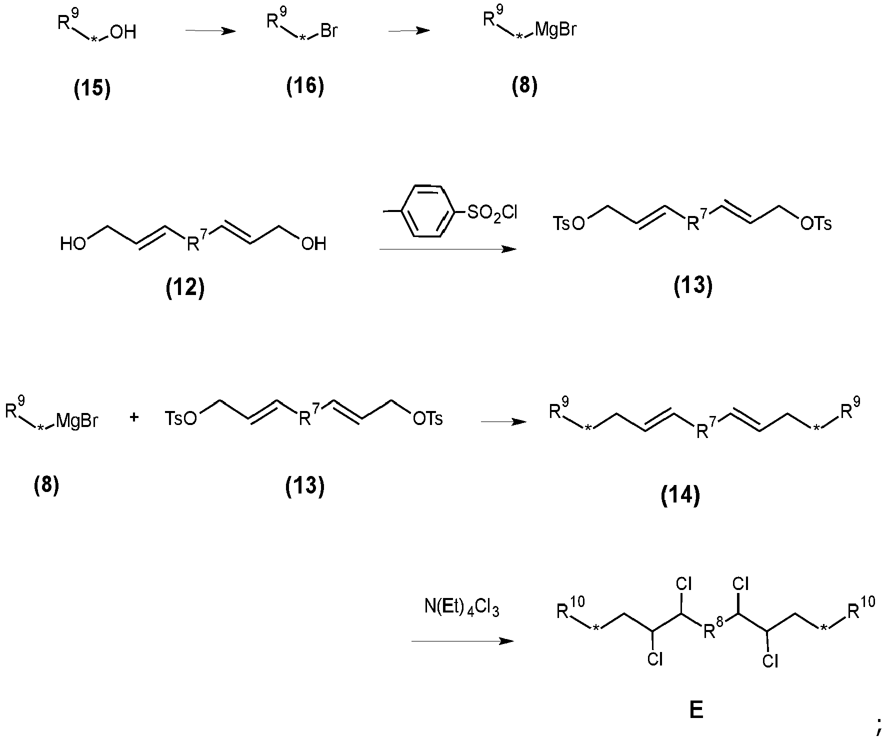 wherein:
- R^{∗} = ¹³Cₐ-n-alkyl,
- ^{∗} = ¹³C; Ph = phenyl; Et = ethyl;
- R¹ = ¹³C_{b}-alkylene-residue, optionally including one or an odd number of chlorine atoms with the proviso that no chlorine is on the terminal end of the residue, and wherein the number of double bonds in the alkylene residue is a minimum of one and up to the theoretically possible number of accumulated double bonds;
- R²= ¹³C_{b}-chloro-alkyl-residue, particularly of the formula ¹³C_{b}-CₙH_{2n+1-y}Cl_{y};
- R³ = ¹³C_{d}-alkylene-residue, optionally including one or an odd number of chlorine atoms with the proviso that no chlorine is on the terminal end of the residue, and wherein the number of double bonds in the alkylene residue is a minimum of one and up to the theoretically possible number of accumulated double bonds;
- R⁴= ¹³C_{d}-chloro-alkyl-residue, particularly of the formula ¹³C_{d}-CₙH_{2n-y}Cl_{y};
- R⁵ = ¹³Cₑ-alkylene-residue, optionally including one or an odd number of chlorine atoms with the proviso that no chlorine is on the terminal end of the residue, and wherein the number of double bonds in the alkylene residue is a minimum of one and up to the theoretically possible number of accumulated double bonds;
- R⁶=¹³Cₑ-chloro-alkyl-residue, particularly of the formula ¹³Cₑ-CₙH_{2n+1-y}Cl_{y};
- R⁷ = ¹³C_{f}-alkylene-residue, optionally including one or an odd number of chlorine atoms, and wherein the number of double bonds in the alkylene residue is a minimum of one and up to the theoretically possible number of accumulated double bonds;
- R⁸= ¹³C_{f}-chloro-Cₙ-alkyl-residue, particularly of formula ¹³C_{f}-CₙH_{2n-y}Cl_{y};
- R⁹= ¹³C_{g}-alkyl or alkylene-residue which can contain 1 double bond;
- R¹⁰ = ¹³C_{g}-(chloro)-alkyl-residue, and is the same as R⁹ if R⁹ =¹³C_{g}-alkyl-residue;
- a, b, c, d, e, f, g = independently from one another an integer of from 1 to the entire number of carbon atoms in the respective alkyl or alkylene residue, preferably each from 1 to 4, and representing the number of ¹³C carbons in the respective residue;
- n is an integer from 0 to 28, preferably from 1 to 28, representing the number of carbon atoms in each of the residues and its value is each time independent from its value in the other residues;
- y is an integer from 0 to 28, preferably from 1 to n-1, representing the number of chlorine atoms in the respective residue.

5. Process according to any one claims 1, 3 or 4, **characterized in that** the alkenes are prepared from unsaturated fatty acids via the following steps:
A) providing an unsaturated fatty acid or a mixture of unsaturated fatty acids,
Ca) converting the unsaturated fatty acid or unsaturated fatty acids to a corresponding alkenol or mixture of alkenols, and
Cb1) reducing the alkenol or mixture of alkenols to the corresponding alkene or mixture of alkenes, or
Cc1) replacing the alcohol group or groups with a leaving group or groups, preferably tosylate,
Cc2) coupling an alkyl to the former alcohol carbon, thereby eliminating the leaving group and forming an unsaturated bond; or
Da) converting the unsaturated fatty acid or unsaturated fatty acids to a corresponding alkenal or mixture of alkenals, and
Db1) coupling the alkenal or mixture of alkenals with Wittig salt to the corresponding alkene or mixture of alkenes.

6. Process for preparing ¹³C-labelled or unlabelled chlorinated paraffins, preferably having neither geminal nor terminal chlorine atoms in their structure, by converting an unsaturated fatty acid or a mixture of unsaturated fatty acids to a ¹³C-labelled or unlabelled chlorinated paraffin or a mixture of ¹³C-labelled or unlabelled chlorinated paraffins, comprising the steps of
A) providing a ¹³C-labelled or unlabelled unsaturated fatty acid or a mixture of ¹³C-labelled or unlabelled unsaturated fatty acids,
Ba) chlorinating the unsaturated fatty acid or unsaturated fatty acids and
Bb) decarboxylating of the resulting chlorinated acid or acids, or
Ca) converting the unsaturated fatty acid or unsaturated fatty acids to a corresponding alkenol or mixture of alkenols, and
Cb1) reducing the alkenol or mixture of alkenols to the corresponding alkene or mixture of alkenes, and
Cb2) chlorinating the resulting alkene or mixture of alkenes; or
Cc1) replacing the alcohol group or groups with a leaving group or groups, preferably tosylate,
Cc2) coupling an alkyl to the former alcohol carbon, thereby eliminating the leaving group and forming an unsaturated bond, and
Cc3) chlorinating the resulting alkene or mixture of alkenes; or
Cd) chlorinating the alkenol or mixture of alkenols; or
Da) converting the unsaturated fatty acid or unsaturated fatty acids to a corresponding alkenal or mixture of alkenals, and
Db1) coupling the alkenal or mixture of alkenals with Wittig salt to form the corresponding alkene or mixture of alkenes,
Db1a) optionally using Wittig salts with at least one ¹³C-labelled carbon atom in the coupling group, and
Db2) chlorinating the resulting alkene or mixture of alkenes.

7. ¹³C-labelled chlorinated paraffins having neither geminal nor terminal chlorine atoms in their structure, optionally prepared according to a process according to any of claims 1 to 6, having
- at least two chlorine atoms in their main chain, and
- at least one, particularly from two to eight, carbon atoms of the main chain replaced by ¹³C-atoms,
preferably represented by the general formula
¹³Cₘ-CₚH₂ₚ₊₂₋ₓClₓ,
with
m being an integer of from 1 to p, particularly 2 to 8;
p being an integer of from 2 to 38, preferably from 8 to 38, particularly 8 to 30;
x = 2 to p-2, preferably 4 to p-2, more preferably, in the case of SCCP 4 to 6, particularly 4 or 6, in the case of MCCP 6 to 8, particularly 6 or 8, in the case of LCCP 8 to 10, particularly 8 or 10.

8. ¹³C-labelled chlorinated paraffins having neither geminal nor terminal chlorine atoms in their structure, optionally according to claim 7, represented by
- either the general formula A 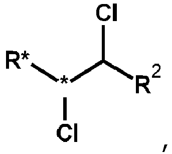
- or general formula B 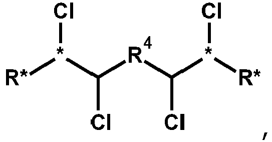
- or general formula D 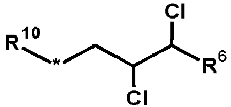
- or general formula E 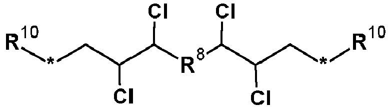 wherein the variables have the meaning defined above in claim 4.

9. ¹³C-labeled chlorinated paraffins having neither geminal nor terminal chlorine atoms in their structure, preferably according to claim 7 or 8,
- of general formula A 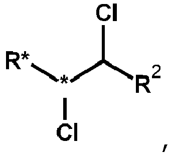 wherein ^{∗} = ¹³C and R^{∗} are as defined above in claim 4, or selected from the group consisting of
i) 6,7,8-¹³C₃-2,3,5,6-tetrachlorooctane or 2,3,5,6-tetrachlorooctane-6,7,8-¹³C₃ of formula 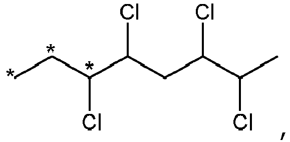
ii) 1,2,9,10-¹³C₄-2,3,8,9-tetrachlorodecane 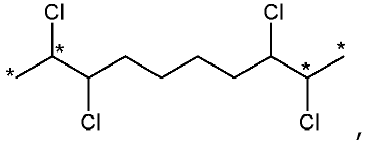
iii) 9,10,11-¹³C₃-2,3,5,6,8,9-hexachloroundecane or 2,3,5,6,8,9-hexachloroundecane-9,10,11-¹³C₃ of formula 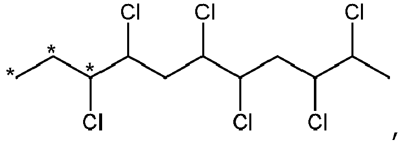
iv) 11,12,13-¹³C₃-3,4,6,7,10,11-hexachlorotridecane or 3,4,6,7,10,11-hexachlorotridecane-11,12,13-¹³C₃ of formula 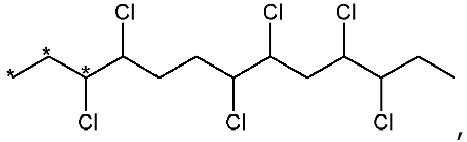
v) 1,2,9,10-¹³C₄-2,3,5,6,8,9-hexachlorodecane-1,2,9,10-¹³C₄ or 2,3,5,6,8,9-hexachlorodecane-1,2,9,10-¹³C₄ of formula 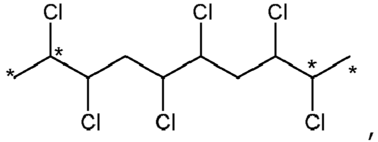
vi) 6,7,8-¹³C₃-2,3,5,6,8,9,11,12-octachlorotridecane or 2,3,5,6,8,9,11,12-octachlorotridecane-6,7,8-¹³C₃ of formula 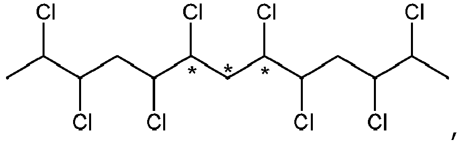
vii) 10,11,12-¹³C₃-2,3,5,6,9,10-hexachlorododecane 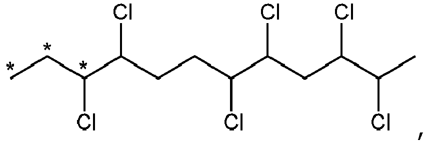 or
viii) 10,11,12-¹³C₃-2,3,5,6,8,9-hexachlorododecane 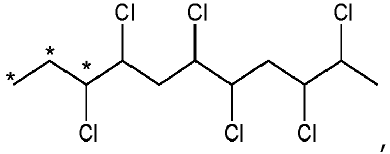
ix) 1,2,3-¹³C₃-3,4,7,8,11,12-hexachlorotetradecane 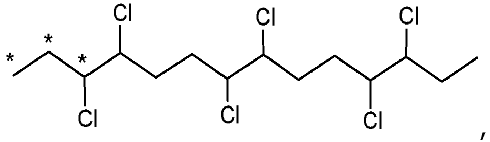
x) 13,14,15-¹³C₃-3,4,7,8,12,13-hexachloropentadecane 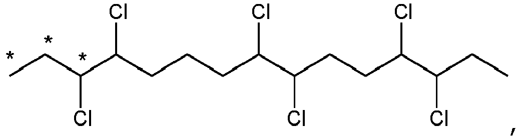
xi) 1,2,3-¹³C₃-3,4,8,9,13,14-hexachlorohexadecane 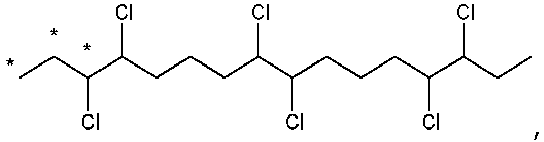
xii) 1,2,3-¹³C₃-3,4,12,13,15,16,18,19-octachloro-n-heneicosane. 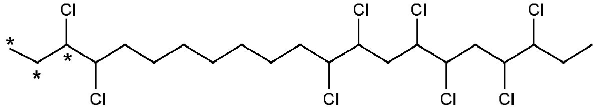 wherein ^{∗} is as defined above.

10. Mixture of chlorinated paraffins comprising ¹³C-labelled chlorinated paraffins having neither geminal nor terminal chlorine atoms in their structure according to any one of claims 7 to 9 or prepared according to any one of claims 1 to 6.

11. Use of ¹³C-labelled chlorinated paraffins having neither geminal nor terminal chlorine atoms in their structure according to any one of claims 7 to 9 or prepared according to any one of claims 1 to 6 as internal standards for analytical processes, in particular for the quantitative analysis of very short-chain chlorinated paraffins (vSCCPs, <C10), short-chain chlorinated paraffins (SCCPs, C10-C13); medium-chain chlorinated paraffins (MCCPs, C14-C17) and long-chain chlorinated paraffins (LCCPs, C18-C30).

12. Use of ¹³C-labelled chlorinated paraffins having neither geminal nor terminal chlorine atoms in their structure according to any one of claims 7 to 9 or prepared according to any one of claims 1 to 6 as reference materials for metabolism and toxicity analysis of chlorinated paraffins.

13. Chlorinated paraffins selected from the group consisting of 9,10,11-¹³C₃-1,2,4,5,8,9-hexachloroundecane, 10,11,12-¹³C₃-1,2,5,6,9,10-hexachlorododecane, 11,12,13-¹³C₃-1,2,6,7,10,11-hexachlorotridecane, 12,13,14-¹³C₃-1,2,7,8,11,12-hexachlorotetradecane, 13,14,15-¹³C₃-1,2,7,8,12,13-hexachloropentadecane, 14,15,16-¹³C₃-1,2,8,9,13,14-hexachlorohexadecane, 1,2,9,10-¹³C₄-2,3,8,9-tetrachlorodecane, 8,9,10-¹³C₃-1,2,4,5,7,8-hexachlorodecane, and mixtures thereof, particularly 1,2,9,10-¹³C₄-2,3,8,9-tetrachlorodecane or 8,9,10-¹³C₃-1,2,4,5,7,8-hexachlorodecane.

14. Internal standards for analytical processes, in particular for the quantitative analysis of vSCCPs, SCCPs, MCCPs, LCCPs, comprising or consisting of
- one or more of the ¹³C-labelled chlorinated paraffins having neither geminal nor terminal chlorine atoms in their structure according to any one of 7 to 9 or prepared according to any one of claims 1 to 6,
- one or more of the chlorinated paraffins of claim 13,
- or a mixture of one or more of the chlorinated paraffins of claim 13 and one or more of the ¹³C-labelled chlorinated paraffins having neither geminal nor terminal chlorine atoms in their structure according to any one of claims 7 to 9 or prepared according to any one of claims 1 to 6.

15. Internal standards for analytical processes according to claim 14, **characterized in that** they comprise or consist of single chain CP mixtures of ¹³Cₘ-CₚH₂ₚ₊₂₋ₓClₓ, with m being an integer of from 1 to p, particularly 2 to 8; p being an integer ≥ 8, particularly 8 to 30; x = 2 to p-1, preferably 2 to 10 or x= 2 to p-2, preferably 4 to p-2; and wherein the ¹³C-labelled single-chain mixtures have chlorine contents of 45-75 weight%, calculated from the analysis on NMR and chlorine titration.
